# EUROPEAN PATENT APPLICATION

(11) **EP 4 316 476 A1**
(43) Date of publication of application: **07.02.2024**
(21) Application number: 22386055.2
(22) Date of filing: 02.08.2022
(51) Int. Cl.: A61K 31/122, A61K 31/69, A61P 35/00

(54) **ANTICANCER AGENTS BASED ON CYCLOPENTENONES**

(71) Applicant: University of Crete, 71003 Heraklion (GR); National and Kapodistrian University of Athens, 10679 Athens (GR); Elipoulos, G. Aristides, 15669 Papagou-Athens (GR)
(72) Inventor: Vassilikogiannakis, Georgios, 71003 Heraklion (GR); Eliopoulos, G. Aristides, 15669 Papagou, Athens (GR); Stratakis, Manolis, 71003 Heraklion (GR); Montagnon, Tamsyn, 71003 Heraklion (GR); Kalaitzakis, Dimitris, 71003 Heraklion (GR)
(74) Representative: Vavekis, Konstantinos

(57) **Abstract**

The invention discloses novel 4-hydroxycyclopent-2-en-1-one derivatives as active compounds in pharmaceutical compositions for the treatment of cancer. The invention confers cytotoxic effects of the said compounds on ovarian, colorectal, cervical, hepatocellular, lung, bladder and breast carcinomas, melanoma, lymphoma and myeloma malignant cells, for the inhibition of cell cycle progression at the G2/M phase, for reducing the expression of DNA replication licensing factors and for having general cancer treatment effects. It further discloses the use of the said compositions for the treatment of platinum-resistant tumors. Another aspect of the invention is the synergetic effects of these compounds with existing cancer treatment medicaments as the proteasomal inhibitors. Finally, the synthetic methods of the active compounds of the said compositions are disclosed.

## Description

### Technical field

The invention relates to a method of treatment of cancer by administering novel cyclopentenone derivatives as active compound in a pharmaceutical composition. The invention confers cytotoxic effects of the said compounds on ovarian, colorectal, cervical, hepatocellular, lung, bladder and breast carcinomas, melanoma, lymphoma and myeloma malignant cells, for the inhibition of cell cycle progression at the G2/M phase, for reducing the expression of DNA replication licensing factors and for having general cancer treatment effects. It also relates to a method of using such compounds in the treatment of abnormal cell growth in mammals, especially humans, and to pharmaceutical compositions as anticancer agents. It further discloses the use of the said compositions for the treatment of platinum-resistant tumors. Another aspect of the invention is the synergetic effects of these compounds with existing cancer treatment medicaments as the proteasomal inhibitors. Finally, the synthetic methods of the active compounds of the said compositions are disclosed.

### STATE OF ART

Cancer is a group of related diseases in which some of the body's cells grow without control and spread to other parts of the body. Treatment of these diseases encompasses surgery, radiotherapy, chemotherapy, immunotherapy and combinations thereof and is important in order to extend patient survival. A chemotherapeutic agent is a chemical molecule that can be used to treat cancer. Examples include, but are not limited to, platinum-based compounds (cisplatin, carboplatin, oxaliplatin), mitomycin C, bleomycin, topotecan, irinotecan, docetaxel, paclitaxel, vincristin, plicamycin, daunorubicin, adriamycin, 5-fluorouracil, and hormone antagonists.

A chemotherapeutic agent can be selected based on the type of cancer being treated, the expression of one or more molecular markers by cancer, the age and general health of the subject to be treated, etc. For example, high grade serous epithelial ovarian cancer (EOC) is usually managed with surgery followed by adjuvant platinum (cis-platin or carboplatin) and taxane combination chemotherapy. Today, one of the most used, well-known chemotherapeutic drug is cis-platin due to its wide range of different types of cancer treatment like carcinomas, germ cell tumors, lymphomas, and sarcomas in different areas of the human body.

However, because of drug resistance and numerous undesirable side effects (i.e. severe kidney problems, allergic reactions, decreased immunity to infections, gastrointestinal disorders, hemorrhage, and hearing loss), several other platinum-containing anti-cancer drugs such as oxaliplatin have also been used as general cancer treatment active chemotherapy drugs. These Platinum-based drugs are available for a wide range of cancer types but have similar problems to cis-platin, including development of resistance. As an example, most EOC patients (approx. 75%) experience disease relapse within the first 5 years despite aggressive treatment at diagnosis. Topotecan and liposomal doxorubicin are currently among second line therapy choices for platinum resistant ovarian cancer with objective response rates of approximately 10% to 15% in platinum-resistant patients with the median progression-free survival of 9.1-13.6 weeks and overall survival of just 35.6-41.3 weeks [1]. Therefore, resistance to platinum-based chemotherapy is a major clinical problem with limited options for management. Other therapeutic agents used in EOC include antiangiogenics and PARP inhibitors which can be administered only to a subset of patients who express particular markers but they have been reported to decrease response to subsequent platinum-based chemotherapy [2].

Another example is cancer of the colon, a leading cause of cancer deaths worldwide. Some drugs commonly used for the management of colorectal cancer include 5-Fluorouracil (5-FU), Capecitabine, a pill that is changed into 5-FU once it gets to the tumor, Irinotecan, Oxaliplatin and their combinations. Sometimes, chemotherapy drugs are given along with a targeted therapy drug. Lonsurf, a fixed-dose combination medication is used as a third- or fourth-line treatment of metastatic colorectal cancer, after chemotherapy and targeted therapeutics have failed.

The prior art therapeutics suffer from the problems of low cytotoxic activity for cancer cells, specific cytotoxic activity for specific cancer types, resistance after some period as, for example, following therapy with cis-platin, and/or lack of ability to prevent cancers bearing translation addiction.

The key advantages of the disclosed compositions, 4-hydroxycyclopent-2-en-1-one derivatives, hereafter referred to as "Enones", compared to the prior art are:
1) Broad spectrum cytotoxic activity due to the high efficacy of Enones against different cancer types in vitro and in vivo.
2) Potential for treatment of platinum-resistant tumors since cis-platin-resistant ovarian cancer cell lines do not display cross-resistance to 4-hydroxy-4-methyl-5-(naphthalen-2-yl)cyclopent-2-en-1-one, (referred as Enone-1) or 5-(4-fluoronaphthalen-1-yl)-4-hydroxy-4-methylcyclopent-2-en-1-one, (referred as Enone-2).
3) Potential for improved therapeutic outcome in tumors possessing elevated expression of replication licensing factors such as CDC6 and CDT1 since Enones-1 and 2 reduce the expression of CDC6 and CDT1 in human tumor cells.
4) Potential for improved therapeutic outcome in tumors possessing "translation addiction" since Enone-1 and Enone-2 are inducers of the p53 pathway which is mediated by ribosomal stress pathways.
5) Potential enhancement of proteasomal inhibitor therapy since Enone-2 synergizes with bortezomib in tumor cell killing.

Although the general type and the synthetic routes for the novel 4-hydroxycyclopent-2-en-1-one derivatives which are the active compounds of the claimed compositions have been published [3, 4], neither 4-hydroxycyclopent-2-en-1-one derivatives with naphthyl group at carbon-5 nor the specific compounds 5-(4-fluoronaphthalen-1-yl)-4-hydroxy-4-methylcyclopent-2-en-1 -one or 4-hydroxy-4-methyl-5-(naphthalen-2-yl)cyclopent-2-en-1-one with the highest cytotoxic activity have ever been reported in the literature nor their specific synthetic routes due to their difficult synthesis because of the steric hindrance of the naphthyl group.

### DESCRIPTION OF THE INVENTION

The present invention discloses a method of treatment using 4-hydroxycyclopent-2-en-1-one derivatives as medicaments and more specifically for a range of types of cancer based on the treatment of the subject with a composition with an active compound (the compound that is mainly responsible for the cytotoxic effect. More specifically the method of treating abnormal cell growth in a mammal (a subject) comprises administering to said mammal a pharmaceutical composition comprising a compound (4-hydroxycyclopent-2-en-1-one derivative) having the structure: 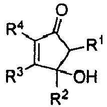 wherein
R¹ is phenyl, substituted phenyl, 2-naphthyl, substituted 1-naphthyl, -CO₂R, -H, alkyl
R² is -H, alkyl, alkenyl, benzyl
R³ is -H, alkyl
R⁴ is -H, alkyl,
or a tautomer, pharmaceutically acceptable salt thereof.

In one embodiment the method comprises administering to a subject a pharmaceutical composition comprising a compound (4-hydroxycyclopent-2-en-1-one derivative) having the above structure wherein
R¹ is phenyl, 4-fluorophenyl, naphthalen-2-yl, 4-fluoronaphthalen-1-yl, CO₂Et, CO₂Me, H, n-butyl
R² is H, methyl, n-C₁₆H₃₃, benzyl, n-pentyl, n-hexyl, pent-4-enyl
R³ is H, methyl,
R⁴ is H, methyl
since the cytotoxic effects of the said group of 4-hydroxycyclopent-2-en-1-one derivatives are higher and preferably to have mainly (more than 50% the trans isomer) trans-stereochemistry so that the 4-hydroxy group is trans to the substituent in position-5 in most of the cases.

In a preferred embodiment, the method comprises administering to a subject a pharmaceutical composition comprising a compound (4-hydroxycyclopent-2-en-1-one derivative) having any of the structures: 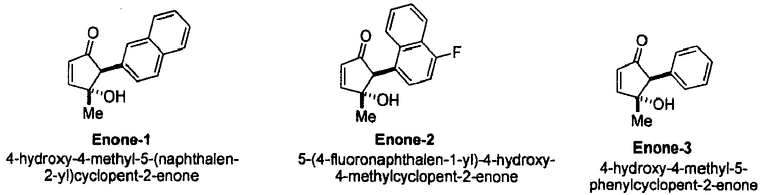 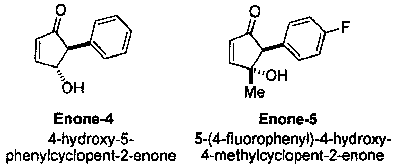 wherein they have mainly (more than 50%) trans-stereochemistry so that the 4-hydroxy group is trans to the substituent in position-5 or combinations thereof, due to their high cytotoxic effects for cancer treatment.

In another embodiment, the method administers to the subject a pharmaceutical composition further comprising a delivery vehicle which enhances cellular uptake and/or stability of the compound in order to be suitable and stable to be used as a medicament.

In another embodiment the method entails administering the said composition in combination with a proteasomal inhibitor and preferably the proteasomal inhibitor bortezomib due to the synergetic effects of these compounds with existing cancer treatment medicaments as the proteasomal inhibitors.

In another aspect of the invention, the method administers the said composition to subjects with malignant cell growth, wherein said malignant cell growth is resistant to platinum-based chemotherapy and preferably to cis-platin treatment and/or oxaliplatin due to its ability to overcome this resistance.

In a further aspect of the invention, the method administers the said composition to subjects with abnormal cell growth, wherein said abnormal cell growth exhibits translational addiction.

In a preferred embodiment the method is used when the said abnormal cell growth is cancer and more preferably the said cancer is selected from the group consisting of liver cancer, lung cancer, pancreatic cancer, cutaneous or intraocular melanoma, uterine cancer, ovarian cancer, rectal cancer, cancer of the anal region, colon cancer, breast cancer, carcinoma of the cervix, cancer of the bladder, lymphoma, myeloma or a combination of one or more of the foregoing cancers.

In another aspect of the invention the compounds acting as the active ingredient of the said compositions used in the above cancer treatment methods have the formula 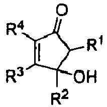 wherein
R¹ is phenyl, 4-fluorophenyl, naphthalen-2-yl, 4-fluoronaphthalen-1-yl, CO₂Et, CO₂Me, H, n-butyl
R² is H, methyl, n-C₁₆H₃₃, benzyl, n-pentyl, n-hexyl, pent-4-enyl
R³ is H, methyl,
R⁴ is H, methyl
and preferably to have mainly (more than 50% the trans isomer) the ones with trans-stereochemistry which means that the 4-hydroxy group is trans to the substituent in position-5 (in most of the cases) or combinations thereof due to the high cytotoxic effects for cancer treatment.

In a further embodiment, we claim the compounds with the formulas 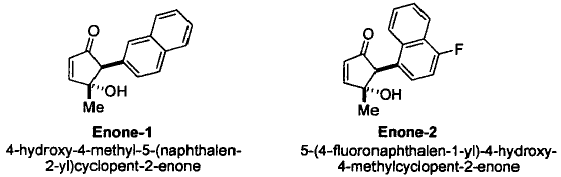 belonging to the above general type, due to their effects for treatment of abnormal cell growth and preferably of cancer and preferably the trans isomer (the 4-hydroxy group is trans to the substituent in position-5).

A third aspect of the present invention relates to the compounds themselves and their synthesis. Neither 4-hydroxycyclopent-2-en-1-one derivatives with naphthyl group at carbon-5 nor the specific compounds 5-(4-fluoronaphthalen-1-yl)-4-hydroxy-4-methylcyclopent-2-en-1-one or 4-hydroxy-4-methyl-5-(naphthalen-2-yl)cyclopent-2-en-1-one (with the highest cytotoxicity) have ever been reported in the literature or disclosed. Also there is no reference to their specific synthetic routes due to their difficult synthesis because of the steric hindrance of the naphthyl group. The published synthetic route for 4-hydroxycyclopent-2-en-1-one derivatives is modified and in particular, the concentration of the photosensitizer, rose Bengal, was increased from 0.0001 M to 0.0015 M and the irradiation time was increased from 3 min to 5 min. This allowed the synthesis of these novel compounds claimed as medicaments.

### BRIEF DESCRIPTION OF THE DRAWINGS

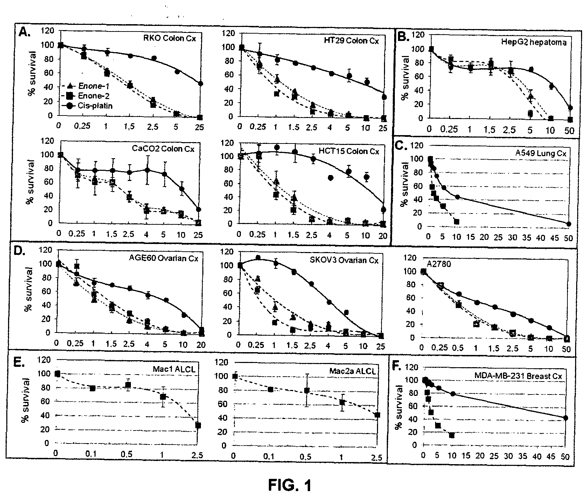

Figure 1: Results of indicative MTT conversion assays showing % survival of cell lines representing different types of malignancies following a 48 hr exposure to increasing concentrations of Enone-1, Enone-2 and/or cis-platin. Cell lines shown include human colorectal carcinoma (Caco2, RKO, HCT15, HT29; 1A), liver (HepG2; 1B), lung (A549; 1C), ovarian carcinoma (A2780, 2780CP, AGE60, SKOV3; 1D), anaplastic large cell lymphoma (Mac1 and Mac2a; 1E) and breast carcinoma (MDA-MB-231; 1F). The x axis represents drug concentrations in µM. The IC50 values of all cell lines tested were calculated from multiple experiments (n≥3) and are provided in Table 1. Grey triangles represent Enone-1; black squares Enone-2, black circles represent cis-platin.

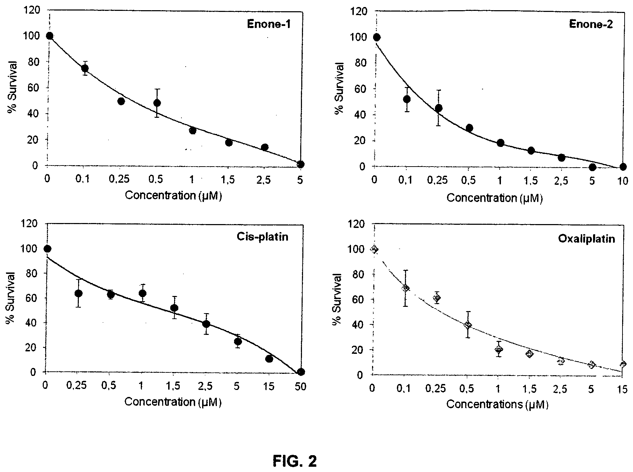

Figure 2: Effects of Enones versus cis-platin and oxaliplatin on A2780 cell survival measured by a PrestoBlue in a 72 hour treatment assay.

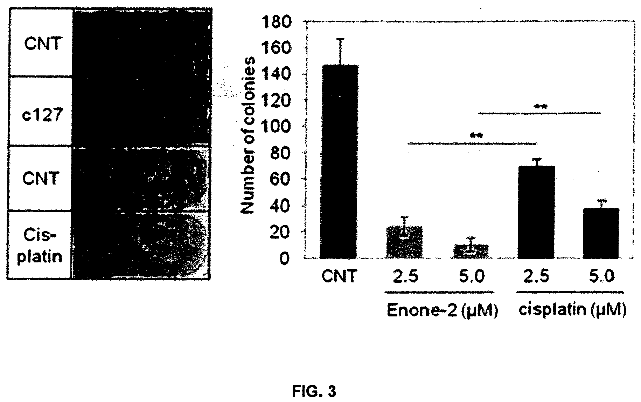

Figure 3: Long-term effects of Enone-2 vs cis-platin on the survival of A2780 cells, measured by formation of colonies that contained more than 100 cells following a 12 hour drug treatment and 10 days of culture in the absence of drug.

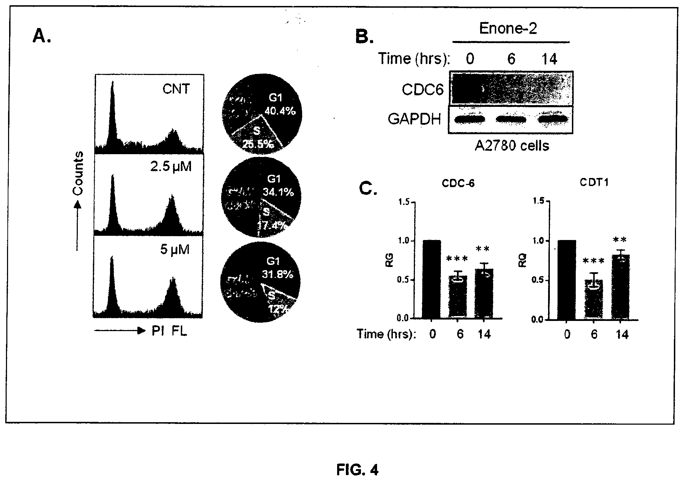

Figure 4. Effects of Enone-2 on cell cycle and on the expression levels of replication licensing factors (RLF). (A) Representative cell cycle profiles showing progressive accumulation of A2780 ovarian carcinoma cells in the G2/M phase of the cell cycle following 24 hour treatment with 2.5 or 5 µM Enone-2. Quantification of results is shown in the form of pie charts on the right. PI: propidium iodide. (B) & (C) A2780 cells were treated with 2.5µM Enone-2 for 6 or 14 hours and analyzed for CDC6 protein levels by immunoblot (B), or RNA levels by reverse transcription qPCR (C). The RNA levels of the RLF CDT1 were also measured by qPCR (C). ***P<0.001; **P<0.01.

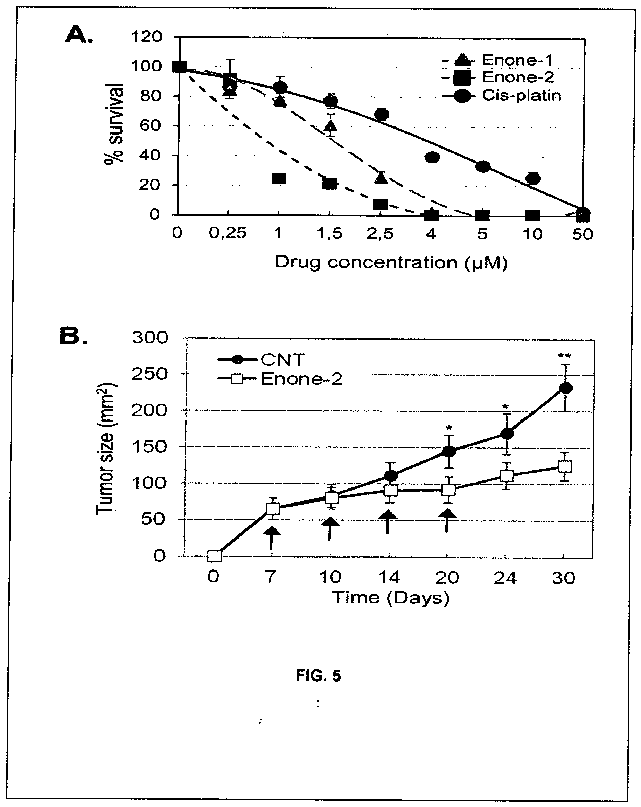

Figure 5: Effects of enone compounds on mouse tumor growth. (A) MB49 mouse bladder carcinoma cells were treated in vitro with increasing concentrations of Enone-1, Enone-2 or cis-platin for 48 hrs and survival was determined by MTT conversion assays compared to untreated control cells. (B) Enone-2, which demonstrated the highest in vitro cytotoxic activity, was used to treat subcutaneous MB49 tumors in C57BL/6 mice. Results show subcutaneous tumor growth following 4 intratumoral injections (arrows) of 5mg/kg Enone-2 vs saline as control.

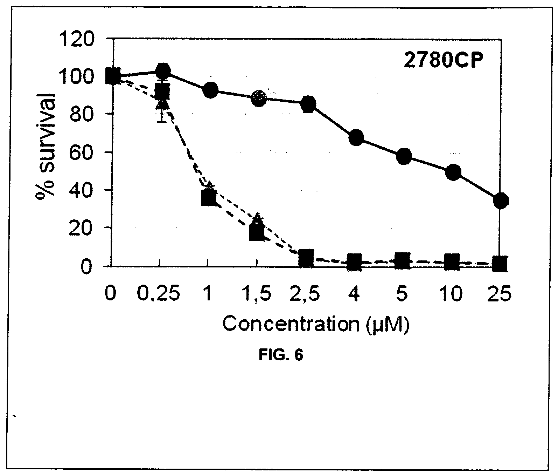

Figure 6: Response of platinum-resistant 2780CP cells to Enone-2. Note that whereas these cells are less sensitive to cis-platin (compare to Fig. 1D), they remain sensitive to Enone1 and Enone-2.

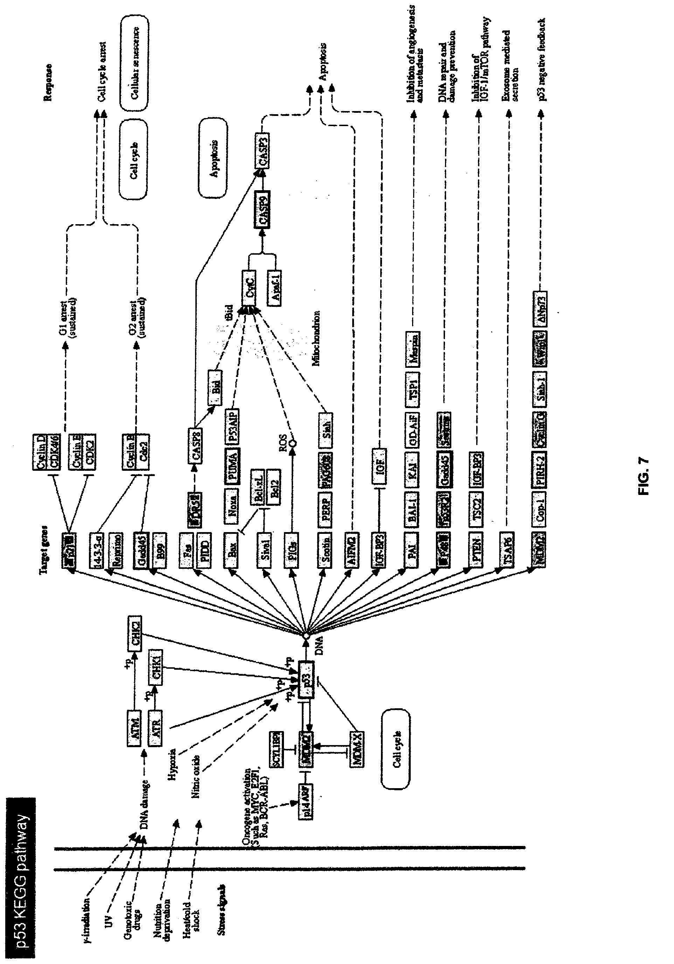

Figure 7: Schematic representation of KEGG p53 pathway-related genes and their cellular roles. Genes found upregulated by RNAseq both at 6 and 14 hrs of Enone-2 treatment are shown in orange boxes. Gene transcripts found upregulated only at 6 hrs or 14 hrs of treatment are denoted with the red and blue frame, respectively.

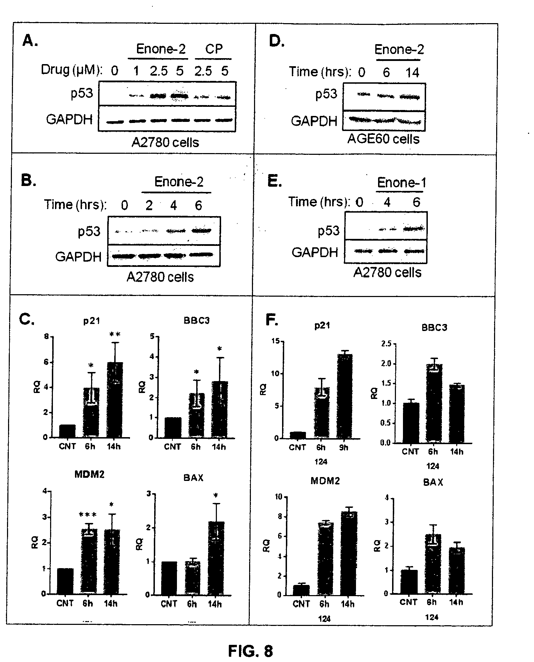

Figure 8: Enones induce the p53 pathway. (A) A2780 cells were cultured for 6 hrs in the presence of increasing concentrations of Enone-2 or cis-platin (CP), as indicated. Cell lysates were analyzed for p53 and GAPDH (loading control). (B) A2780 cells were cultured in the presence or absence of 2.5µM for the indicated time points before being analyzed for p53 and GAPDH levels. (C) Treatment of A2780 cells with 2.5µM Enone-2 results in the up-regulation of p53-regulated genes (*p<0.05; **p<0.01; ***p<0.001, n=4). (D) Induction of p53 accumulation in AGE60 ovarian carcinoma cells following 6 or 14 hr exposure to 2.5µM Enone-2. (E) Enone-1 induces the p53 pathway in A2780 cells which were cultured in the presence of 2.5µM of the drug and analyzed as in (B). (F) Treatment of A2780 cells with 2.5µM Enone-2 results in the up-regulation of p53-regulated genes (*p<0.05; **p<0.01; ***p<0.001, n=3).

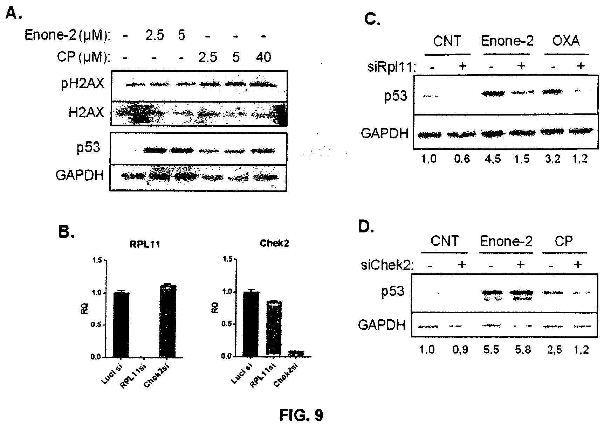

Figure 9. (A) Effects of Enone-2 and cis-platin on yH2Ax (pH2Ax), a marker of DNA damage response, versus H2Ax as loading control and on p53 levels. A2780 cells were cultured for 6 hrs in the presence of drug concentrations as indicated. (B) Effects of siRNA targeting RPL11 (siRPL11), Chek2 (siChek2) or the unrelated luciferase (Luci) genes on RPL11 and Chek2 mRNA levels quantified by RT-PCR. (C) Effects of siRpl11 on Enone-2 and oxaliplatin (OXA)-induced p53 activation. Expression levels relative to GAPDH are indicated; untreated controls (CNT) were given the arbitrary value of 1. (D) Effects of siChek2 on Enone-2 and cis-platin (CP)-induced p53 activation. Expression levels relative to GAPDH are indicated; untreated controls (CNT) were given the arbitrary value of 1.

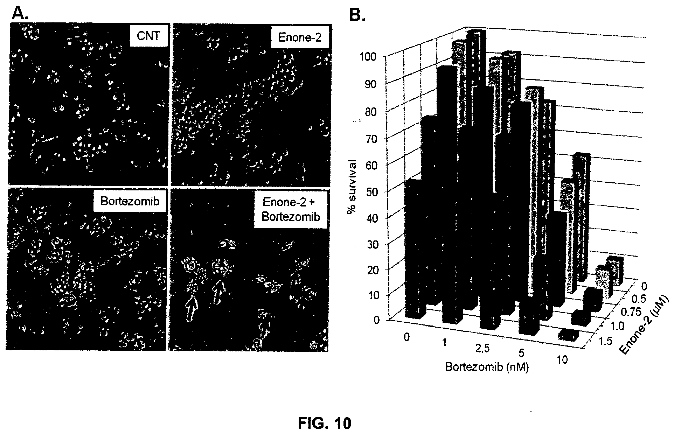

Figure 10: The combination of Bortezomib and Enone-2 results in higher cytotoxicity in relation to each agent alone. The arrows in the microphotographs of (A) depict 2780CP cells with apoptotic morphology following treatment with 5nM bortezomib in combination with 0.5µM Enone-2.

### EXAMPLES

### Synthesis of 4-hydroxy-4-methyl-5-(naphthalen-2-yl)cyclopent-2-en-1-one (2, Enone-1)

### Step 1, Synthesis of 2-methyl-5-(naphthalen-2-ylmethyl)furan (1)

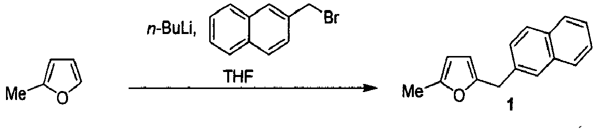

To a solution of 2-methylfuran (670 µL, 7.46 mmol) in anhydrous THF (8 mL), under an argon atmosphere and at 0 °C, a solution of *n*-BuLi (1.6 M in hexane, 4.24 mL, 6.78 mmol) was added dropwise. The solution was stirred for a further 30 min at the same temperature. Afterwards, a solution of the 2-(bromomethyl)naphthalene (750 mg, 3.4 mmol) in anhydrous THF (4 mL) was added dropwise at 0 °C. The solution was then warmed to room temperature and stirred for 3 h. After completion of the reaction, as indicated by tic analysis (3 h), the reaction was quenched with a saturated aqueous solution of NH₄Cl (10 mL) and the resulting mixture was extracted with Et₂O (20 mL). The organic layer was separated, dried over Na₂SO₄ and concentrated *in vacuo.* The product was purified by flash column chromatography (silica gel, petroleum ether) to afford 1 as a slightly yellow oil (yield = 560 mg, 74%).

### Characteristic NMR spectrum

¹H NMR (500 MHz, CDCl₃): 7.81 (m, 3H), 7.70 (s, 1H), 7.46 (m, 2H), 7.40 (dd, *J₁*=8.4 Hz, *J₂*=2.0 Hz, 1H), 5.92 (d, *J*=2.6 Hz, 1H), 5.90 (m, 1H), 4.10 (s, 2H), 2.27 (s, 3H) ppm; ¹³C NMR (125 MHz, CDCl₃): 152.6, 151.1, 135.9, 133.6, 132.2, 128.0, 127.6 (2C), 127.3, 127.0, 125.9, 125.4, 107.0, 106.0, 34.7, 13.5 ppm.

### Step 2 - Synthesis of 4-hydroxy-4-methyl-5-(naphthalen-2-yl)cyclopent-2-en-1-one (2, Enone-1)

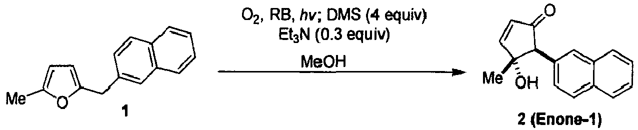

Furan **1** (111 mg, 0.5 mmol) was dissolved in MeOH (5 mL, 0.1. M, in a test tube) containing catalytic amount (7.3 mg, 1.5% mol) of rose Bengal as photosensitizer. The solution was cooled with an ice bath. Oxygen was gently bubbled through the solution while it was irradiated with a xenon Variac Eimac Cermax 300 W lamp. After completion of the reaction (5 min, indicated by tlc), the solution was warmed to room temperature and Me₂S (146 µL, 2 mmol) was added. After completion of the reduction, as was indicated by tlc analysis (40 min), Et₃N (21 µL, 0.15 mmol) was added and the reaction was stirred at rt for 30 min. The reaction was monitored by tlc analysis and by ¹H-NMR. After completion of the reaction (30 min) the solvent was removed *in vacuo* and the residue was purified by flash column chromatography (silica gel, petroleum ether : EtOAc = 3:1 → 2:1) to furnish **2** as a white solid (yield = 103 mg, 87%). For scaling up the process, the 5 min irradiation step was repeated 10 times, at the scale reported above and the photooxidized solutions were combined and subsequently treated with Me₂S and Et₃N.

### Characteristic NMR spectrum

¹H NMR (500 MHz, CDCl₃): 7.80 (m, 3H), 7.60 (s, 1H), 7.47 (m, 3H), 7.16 (dd, *J₁*=8.4 Hz, *J₂*=1.5 Hz, 1H), 6.26 (d, *J*=5.8 Hz, 1H), 3.94 (s, 1H), 1.03 (s, 3H) ppm; ¹³C NMR (125 MHz, CDCl₃): 206.3, 165.9, 133.3, 132.7, 132.5, 132.1, 129.0, 128.1, 127.8, 127.6, 127.4, 126.1, 125.9, 80.4, 65.5, 25.7 ppm.

HRMS (Orbitrap ESI): [M+H]⁺ calcd for C₁₆H₁₅O₂, 239.1067; found, 239.1067.

### Representative NOE for compound 2

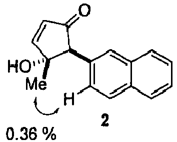

### Synthesis of 5-(4-fluoronaphthalen-1-yl)-4-hydroxy-4-methylcyclopent-2-en-1-one (7, Enone-2)

### Step 1: Synthesis of methyl 4-fluoro-1-naphthoate (3)

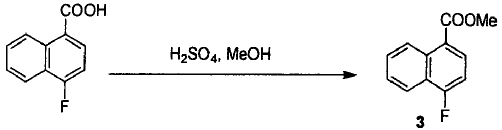

4-Fluoro-1-naphthoic acid (580 mg, 3.05 mmol) was dissolved in MeOH (20 mL, 0.15 M) containing sulfuric acid (160 µL, 3 mmol). The solution was heated overnight to reflux with an oil bath until the full consumption of the starting compound. The mixture was quenched with a saturated aqueous solution of NH₄Cl (15 mL) and the resulting mixture was extracted with Et₂O (30 mL). The organic layer was separated, dried over Na₂SO₄ and concentrated *in vacuo.* The product **3** was used in the next step without further purification (yield = 550 mg, 88%).

### Characteristic NMR spectrum

¹H NMR (500 MHz, CDCl₃): 9.01 (d, *J*=8.8 Hz, 1H), 8.22 (dd, *J₁*=8.2 Hz, *J₂*=5.7 Hz, 1H), 8.17 (d, *J*=8.3 Hz, 1H), 7.68 (m, 1H), 7.61 (t, *J*=7.6 Hz, 1H), 7.16 (dd, *J₁*=9.7 Hz, *J₂*=8.3 Hz, 1H), 4.00 (s, 3H) ppm; ¹³C NMR (125 MHz, CDCl₃): 167.0, 161.6 (d, *J* = 258 Hz), 133.2 (d, *J*=5.4 Hz), 131.3 (d, *J*=9.8 Hz), 128.7, 126.4 (d, *J*=1.8 Hz), 125.9 (d, *J*=2.1 Hz), 123.8 (d, *J*=15.5 Hz), 122.9 (d, *J*=4.3 Hz), 120.8 (d, *J*=6.4 Hz), 108.3 (d, *J*=20.7 Hz), 52.1 ppm.

HRMS (Orbitrap ESI): [M+H]⁺ calcd for C₁₂H₁₀FO₂, 205.0659; found, 205.0658.

### Step 2 - Synthesis of (4-fluoronaphthalen-1-yl)methanol (4)

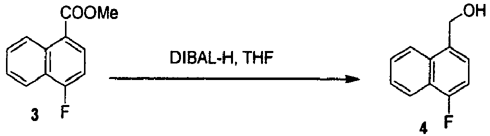

To a stirred solution of the ester **3** (550 mg, 2.70 mmol) in dry THF (10 mL), at -78 °C and under an argon atmosphere, DIBAL-H (1.0 M solution in hexane, 6.75 mL, 6.75 mmol) was added dropwise. The solution was warmed to room temperature and stirred for 1 h. The full consumption of the starting ester was confirmed by tlc analysis. Then the solution was poured directly into a saturated aqueous potassium sodium tartrate solution (10 mL) and stirred for 1 h at rt. The layers were separated and the aqueous layer was extracted with Et₂O (2× 10 mL). The combined organic layers were dried over Na₂SO₄ and concentrated *in vacuo.* The product **4** was used in the next step without further purification (yield = 456 mg, 96%).

### Characteristic NMR spectrum

¹H NMR (500 MHz, CDCl₃): 8.13 (m, 1H), 7.98 (m, 1H), 7.54 (m, 2H), 7.29 (dd, *J₁*=7.8 Hz, *J₂*=5.5 Hz, 1H), 7.02 (dd, *J₁*=10.4 Hz, *J₂*=7.8 Hz, 1H), 4.91 (s, 2H), 3.00 (brs, 1H) ppm; ¹³C NMR (125 MHz, CDCl₃): 158.6 (d, *J*=250.4 Hz), 132.3 (d, *J*=4.7 Hz), 132.0 (d, *J*=4.1 Hz), 127.0, 126.0 (d, *J*=1.5 Hz), 125.1 (d, *J*=8.6 Hz), 123.8 (d, *J*=16.2 Hz), 123.5 (d, *J*=2.7 Hz), 121.0 (d, *J*=5.6 Hz), 108.5 (d, *J*=19.8 Hz), 62.7 ppm.

HRMS (Orbitrap ESI): [M+H]⁺ calcd for C₁₁H₁₀FO, 177.0710; found, 177.0712.

### Step 3 - Synthesis of 1-(bromomethyl)-4-fluoronaphthalene (5)

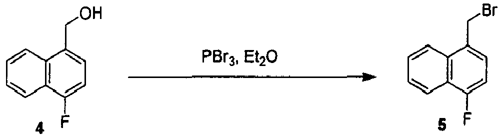

To a stirred solution of the alcohol **4** (456 mg, 2.59 mmol) in dry Et₂O (11 mL), at 0 °C and under an argon atmosphere, was added dropwise PBr₃ (110 µL, 1.16 mmol). The solution was warmed to room temperature and stirred for 30 min. The full consumption of the starting alcohol was confirmed by tlc analysis. The solution was cooled to 0 °C using an ice bath and saturated aqueous solution of NaHCO₃ (10 mL) was added dropwise. The layers were separated and the aqueous layer was extracted with Et₂O (2× 10 mL). The combined organic layers were dried over Na₂SO₄ and concentrated *in vacuo.* The product **5** was used in the next step without further purification (yield = 530 mg, 86%).

### Characteristic NMR spectrum

¹H NMR (500 MHz, CDCl₃): 8.18 (d, *J*=8.6 Hz, 1H), 8.15 (d, *J*=8.6 Hz, 1H), 7.69 (m, 1H), 7.61 (t, *J*=7.8 Hz, 1H), 7.48 (dd, *J₁*=7.8 Hz, *J₂*=5.4 Hz, 1H), 7.08 (dd, *J₁*=10.1 Hz, *J₂*=7.8 Hz, 1H), 4.93 (s, 2H) ppm; ¹³C NMR (125 MHz, CDCl₃): 159.3 (d, *J*=253.4 Hz), 132.3 (d, *J*=5.0 Hz), 129.2 (d, *J*=4.5 Hz), 127.7 (d, *J*=9.0 Hz), 127.5, 126.5 (d, *J*=1.5 Hz), 124.2 (d, *J*=16.4 Hz), 123.8 (d, *J*=2.6 Hz), 121.3 (d, *J*=5.6 Hz), 108.9 (d, *J*=20.1 Hz), 31.2 ppm.

### Step 4 - Synthesis of 2-((4-fluoronaphthalen-1-yl)methyl)-5-methylfuran (6)

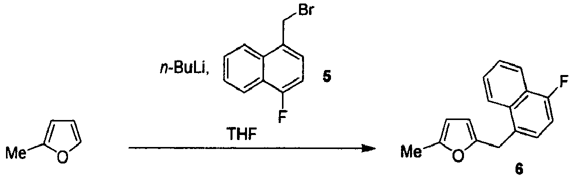

To a solution of 2-methylfuran (800 µL, 8.92 mmol) in anhydrous THF (12 mL), under an argon atmosphere and at 0 °C, was added dropwise a solution of *n*-BuLi (1.6 M in hexane, 4.2 mL, 6.72 mmol). The solution was stirred for a further 30 min at the same temperature. Afterwards, a solution of compound **5** (530 mg, 2.23 mmol) in anhydrous THF (4 mL) was added slowly at 0 °C. The solution was then warmed to room temperature and stirred for 30 min. After completion of the reaction, as indicated by tlc analysis (30 min), the reaction was quenched with a saturated aqueous solution of NH₄Cl (10 mL) and the resulting mixture was extracted with Et₂O (20 mL). The organic layer was separated, dried over Na₂SO₄ and concentrated *in vacuo.* The product was purified by flash column chromatography (silica gel, petroleum ether: EtOAc = 40:1) to afford **6** as a slightly yellow oil (yield = 487 mg, 91%).

### Characteristic NMR spectrum

¹H NMR (500 MHz, CDCl₃): 8.14 (m, 1H), 8.03 (m, 1H), 7.55 (m, 2H), 7.26 (m, 1H), 7.08 (dd, *J₁*=10.3 Hz, *J₂*=7.8 Hz, 1H), 5.84 (d, *J*=2.7 Hz, 1H), 5.76 (d, *J*=2.7 Hz, 1H), 4.32 (s, 2H), 2.26 (s, 3H) ppm; ¹³C NMR (125 MHz, CDCl₃): 158.0 (d, *J*=249.1 Hz), 152.0, 150.8, 133.0 (d, *J*=4.2 Hz), 130.1 (d, *J*=4.5 Hz), 126.8, 126.4 (d, *J*=8.2 Hz), 125.8, 124.1 (d, *J*=2.5 Hz), 124.0 (d, *J*=17.0 Hz), 121.1 (d, *J*=5.5 Hz), 108.9 (d, *J*=19.5 Hz), 107.3, 106.1, 31.6, 13.5 ppm.

HRMS (Orbitrap ESI): [M+H]⁺ calcd for C₁₆H₁₄FO, 241.1023; found, 241.1024.

### Step 5 - Synthesis of 5-(4-fluoronaphthalen-1-yl)-4-hydroxy-4-methylcyclopent-2-en-1-one (7, Enone-2)

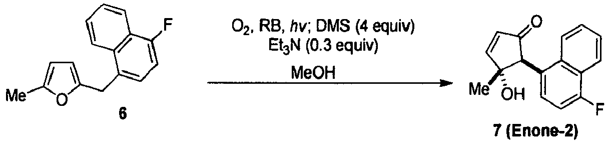

Furan **6** (120 mg, 0.5 mmol) was dissolved in MeOH (5 mL, 0.1 M) containing catalytic amount (7.3 mg, 1.5% mol) of rose Bengal as photosensitizer. The solution was cooled with an ice bath. Oxygen was gently bubbled through the solution while it was irradiated with a xenon Variac Eimac Cermax 300 W lamp. After completion of the reaction (5 min, indicated by tlc), the solution was warmed to room temperature and Me₂S (146 µL, 2 mmol) was added. After completion of the reduction, as was indicated by tlc analysis (40 min), Et₃N (21 µL, 0.15 mmol) was added and the reaction was stirred at rt for 30 min. The reaction was monitored by tlc analysis and by ¹H-NMR. After completion of the reaction (30 min) the solvent was removed *in vacuo* and the residue was purified by flash column chromatography (silica gel, petroleum ether : EtOAc = 7:1 → 4:1) to furnish 7 as a white solid (yield = 85 mg, 66%). For scaling up the process, the 5 min irradiation step was repeated 10 times, at the scale reported above, and the photooxidized solutions were combined and subsequently treated with Me₂S and Et₃N.

### Characteristic NMR spectrum

¹H NMR (500 MHz, CDCl₃): 8.20 (brs, 1H), 8.15 (m, 1H), 7.56 (m, 2H), 7.50 (d, *J*=5.6 Hz, 1H), 7.10 (dd, *J₁*=9.8 Hz, *J₂*=8.3 Hz, 1H), 6.97 (brs, 1H), 6.32 (d, *J*=5.6 Hz, 1H), 4.50 (s, 1H), 2.59 (s, 1H), 0.95 (s, 3H) ppm; ¹³C NMR (125 MHz, CDCl₃): 207.3, 165.9, 158.1 (d, *J*=250.3 Hz), 134.8, 132.3, 128.5 (d, *J*=3.9 Hz), 127.4, 126.6 (d, *J*=8.1 Hz), 126.1, 124.1, 124.0 (d, *J*=17.0 Hz), 121.2 (d, *J*=4.7 Hz), 108.9 (d, *J*=20.0 Hz), 80.9, 60.1, 25.2 ppm.

HRMS (Orbitrap ESI): [M+H]⁺ calcd for C₁₆H₁₄FO₂, 257.0972; found, 257.0969.

### Experiment to prove the Enone-1 and Enone-2 cytotoxic effects on malignant cells

The experiments aim to prove the cytotoxic effects of the final products of the chemical synthesis, 4-hydroxy-4-methyl-5-(naphthalen-2-yl)cyclopent-2-enone (Enone-1) and 5-(4-fluoronaphthalen-1-yl)-4-hydroxy-4-methylcyclopent-2-enone (Enone-2) on ovarian, colorectal, cervical, hepatocellular, lung, bladder and breast carcinomas, melanoma, lymphoma and myeloma malignant cells.

In particular, Enone-1 and Enone-2 were tested for cytotoxic activity in 17 cell lines representing a broad spectrum of human tumors. They include human colorectal carcinoma (Caco2, RKO, HCT15, HT29), liver (HepG2), lung (A549), ovarian carcinoma (A2780, 2780CP, AGE60, SKOV3), anaplastic large cell lymphoma (Mac1 and Mac2a), breast carcinoma (MDA-MB-231), cervical (HeLa), bladder cancer (VM-CUB-1), melanoma (BRO), and multiple myeloma (MR20).

Typically, 7000-8000 cancer cells were plated in 96 well flat-bottom plates in standard growth medium and 16 hrs later the cells were treated with increasing concentrations of Enone-1, Enone-2 or, as a comparison, the established chemotherapeutic agent cis-platin for 48 hrs before cell survival was assessed by using the MTT conversion assay [1]. Each assay was performed in triplicate and repeated in 2-3 independent experiments. Specifically, to measure cell survival, 20 µl of 10 mg/ml MTT (3-(4,4-dimethylthiazol-2-yl)-2,5-diphenyl tetrazolium bromide; Sigma) was added to each well and the plates were incubated at 37°C in 5% CO2 for 3 hours. The supernatant was removed and the formed crystals were dissolved in 200 µl dimethyl sulphoxide. The plates were then analysed at 450 nm on a plate reader. Growth inhibition (reduction in survival) was calculated by expressing the differences in optical densities between treatment wells and control wells as a percentage of the control.

Representative results of MTT conversion assays are shown in Figure 1 and collective data from 3 independent experiments in Table 1. Both Enone-1 and Enone-2 yielded significant cytotoxic effects that superseded those of cis-platin. There was little in-between differences in inhibitory concentrations 50% (IC50) of Enone-1 and Enone-2 in each cell line tested; IC50 values ranged from approx. 0.05 µM in MR20 multiple myeloma cells (most sensitive) to 6.2 µM in HeLa cervical carcinoma cells (less sensitive). As a comparison, the IC50 values for cis-platin were approximately 5.8 µM for MR20 and 31 µM for HeLa cells. Therefore, depending on the cell line, Enones 1 and 2 display 2.5 - 50-fold higher toxicity than cis-platin in a 48 hour exposure assay measured by MTT conversion.

**Table 1: IC50 values for Enone-1, Enone-2 and/or cis-patin in malignant cell lines calculated by MTT conversion assays.**

| **Cell line** | **Tumor Origin** | **IC₅₀ (µM)** | | |
|---|---|---|---|---|
| | | **Enone-1** | **Enone-2** | **Cis-platin** |
| A2780 | Ovarian | 0.6 (±0.15) | 0.5 (±0.1) | 1.4 (±0.2) |
| 2780CP | Ovarian | 0.75 (±0.2) | 0.7 (±0.3) | 10 (±1.5) |
| SKOV3 | Ovarian | 0.8 (±0.15) | 0.3 (±0.1) | 4.0 (±0.8) |
| AGE60 | Ovarian | 1.0 (±0.2) | 1.2 (±0.2) | 5.0 (±1.1) |
| RKO | Colon | 1.3 (±0.3) | 1.2 (±0.2) | 19 (±1.8) |
| HT29 | Colon | 1.2 (±0.1) | 0.9 (±0.15) | 13.5 (±2.2) |
| Caco-2 | Colon | 1.8 (±0.3) | 1.8 (±0.2) | 10 (±0.7) |
| HCT15 | Colon | 1.2 (±0.1) | 0.9 (±0.1) | 14 (±1.2) |
| HepG2 | Liver | 3.8 (±0.2) | 3 (±0.2) | 9.0 (±0.8) |
| A549 . | Lung | 1.7 (±0.2) | 1.5 (±0.2) | 7.5 (±1.2) |
| MDA-MB-231 | Breast | ND | 2.5 (±0.8) | 32 (±3.2) |
| Mac1 | ALCL | ND | 1.8 (±0.2) | ND |
| Mac2a | ALCL | ND | 2.3 (±0.2) | ND |
| MR20 | MM | 0.05 (±0.01) | 0.1 (±0.02) | 5.8 (±0.45) |
| HeLa | cervical | ND | 6.2 ((±1.1) | 31 (±3.3) |
| VM-CUB-1 | bladder | 1.2 (±0.15) | 0.8 (±0.1) | 7.9 (±1.1) |
| BRO - | melanoma | 3.9 (±0.6) . | 3.8 (±0.7) | 15.2 (±2.1) |
| *ALCL: anaplastic large cell lymphoma; MM: multiple myeloma; ND: not determined* | | | | |

The effects of Enone-1 and Enone-2 on cancer cells were confirmed using a second methodology, namely the PrestoBlue assay (PrestoBlue^{™} Cell Viability Reagent, Thermo Fisher, Catalog number: A13261), a cell permeable resazurin-based solution that functions as a cell viability indicator. A2780 ovarian cancer cells were plated as described above for the MTT assays and Enone drugs were added at increasing concentrations as indicated in Figure 2. Cell viability was determined 72 hrs later following the addition of PrestoBlue, according to the manufacturer's instructions (Thermo Fisher). For comparison, the effects of 2 platinum-based chemotherapeutic agents, cis-platin and oxaliplatin, were tested in parallel.

All drugs demonstrated a concentration-dependent reduction in PrestoBlue fluorescence, indicative of reduced A2780 viability (Figure 2). The IC50 values of Enone-1 and Enone-2 using this experimental setting were 0.20 µM (±0.06) and 0.12 µM (±0.07), respectively, the IC50 of cis-platin was 1.4 µM (±0.12) and the IC50 of oxaliplatin was 0.27 µM (±0.03) from n=3 experiments. Therefore, Enone-1 and Enone-2 induced approximately 8 - 13-fold higher cytotoxicity than cis-platin and 1.25 - 2 fold higher cancer cell killing efficacy than oxaliplatin in A2780 cells, measured by PrestoBlue assay.

The long-term effects of Enone-2 on tumor cell survival were examined by using clonogenic assays which assess the ability of single cells to survive and reproduce to form colonies. A2780 ovarian carcinoma cells were treated for 12 hours with 2.5 µM or 5.0 µM Enone-2 or cis-platin, were then washed with PBS to remove excess of drugs, detached by trypsinization, counted and plated in a 6-well tissue culture plate (Costar) at 600 cells per well. The colonies formed on day 10 were visualized by staining with crystal violet following cell fixation and enumerated. Under these conditions, the number of colonies formed by A2780 cells treated with Enone-2 was lower than the equivalent dose of cis-platin (Figure 3).

Therefore, Enone-1 and Enone-2 confer potent cytotoxic effects in several cancer cell lines, superseding those of the established chemotherapeutic agents cis-platin and oxaliplatin.

**Experiments to prove that Enone-1 and Enone-2 suppress cancer cell proliferation by inhibiting cell cycle progression at the G2/M phase and by reducing the expression of DNA replication licensing factors.**

The effect of Enone-2 on cell cycle progression was tested to determine if it causes cell cycle arrest, in addition to inducing cell death. To this end, A2780 ovarian carcinoma cells were treated with 2.5 µM or 5 µM Enone-2 for 24 hours, cells were detached, fixed in 1% Neutral Buffered Formalin and stained with 5 µg/ml propidium iodide. Cell suspensions were next analyzed for fluorescence intensities (DNA content) by quantitative flow cytometry. As shown in Figure 4A, Enone-2 led to accumulation of cells in the G2/M phase of the cell cycle in a concentration dependent manner.

Replication licensing factors (RLF) such as CDC6, MCM2-MCM7, ORC and CDT1 form origins of DNA replication in the G1 phase of the cell cycle and initiate DNA replication during the S phase. CDC6 is also involved in S-M checkpoint mechanisms that ensure that the entire genome is replicated just once per cell division. Cancer cells frequently display deregulated expression of RLFs; for example, CDC6 is over-expressed in colorectal and ovarian carcinoma and is associated with poor patient prognosis [5, 6]. Higher levels of CDC6 are also observed in cis-platin-resistant tumor cell lines and contribute to resistance to both cis-platin and oxaliplatin [7, 8]. Because of these characteristics, RLFs are considered as novel targets for anti-cancer therapy [9]. Enone-2 was found to reduce the key RLF CDC6 in a time-dependent manner at both RNA and protein levels (Figure 4B and 4C) and to also reduce CDT1 RNA levels. We thus claim that Enones target molecular pathways that lead to reduced expression of CDC6 and other RLFs, and induce arrest at the G2/M phase of the cell cycle. The anti-cancer effects of Enone-1 and Enone-2 may thus be particularly relevant to cancers which over-express RLFs.

**Experiment to prove that Enone-2 suppresses tumor growth in vivo.**

The in vivo anti-tumor effect of Enones was determined by xenotransplantation of MB49 mouse bladder carcinoma cells in syngeneic, immunocompetent C57BL/6 female mice.

First, the impact of Enone-1 and Enone-2 on the survival of MB49 cells was verified by MTT assays performed as described in Figure 1. As shown in Figure 5A, the IC50 for Enone-2 was found to be 0.6 µM (±0.1), for Enone-1 was 1.4 µM (±0.2) and for cis-platin, 3.8 µM (±0.3).

On this basis, the *in vivo* anti-tumor activity of Enone-2 was tested. Female mice were transplanted subcutaneously with 10⁶ MB49 cells which formed tumors of approx. 40-60 mm² size by day 7. Mice were randomly separated in 2 groups of 5 animals each and injected intratumorally with 50 µl Enone-2 to achieve 5 mg/kg drug dose on days 7, 10, 14 and 20. The tumor size was monitored using a digital caliper and expressed in mm² by multiplying the length (the longest diameter) with the width (the shortest diameter perpendicular to length) of the tumor. As shown in Figure 5, Enone-2 arrested tumor growth whereas MB49 tumors receiving vehicle control (VC, 50µl saline) continued to grow.

**Experiment to prove that Enone-1 and Enone-2 overcome resistance to cis-platin in vitro.**

Resistance to platinum-based chemotherapy is a major problem in oncology that impacts therapeutic options and limits patient survival. This is relevant to several types of malignancy, including ovarian cancer where platinum resistance frequently occurs. As Enone-1 and 2 show efficacy in reducing survival of A2780 ovarian carcinoma cells, the drugs were also tested in 2780CP, a cis-platin-resistant derivative of A2780 [10]. These cells display approx. 7-fold lower sensitivity to cis-platin compared to A2780 cells (Figure 6 and Figure 1D). Nevertheless, 2780CP cells were found to be nearly as sensitive to Enone-1 and Enone-2 as the cis-platin-sensitive A2780 cells by MTT conversion assays.

We thus claim that cis-platin resistant cells do not to confer cross-resistance to Enones and that Enone-1 and Enone-2 could provide new therapeutic options in platinum-resistant tumors.

**Experiment to prove that Enone-2 is an inducer of ribosomal biogenesis stress.**

Gene expression profiling of A2780 ovarian carcinoma cells exposed to 2.5µM Enone-2 for 6 hrs or 14 hrs disclosed activation of the p53 pathway by virtue of upregulation of several p53 target genes (Figure 7). Activation of p53 occurs in response to DNA damage (so called "DNA damage response", DDR) or ribosomal stress (RS) and conveys signals that arrest cell cycle and induce cell death.

The effects of Enone-2 on p53 and p53-dependent transcription of cell cycle and cell death-associated genes was confirmed by immunoblotting for p53 and by reverse transcription qPCR (RT-qPCR) for *mdm2*, *p21*, *bax* and *bbc3*/*puma*, established p53 target genes. As shown in Figure 8, treatment of A2780 or AGE60 ovarian carcinoma cells with 2.5µM Enone-2 resulted in robust p53 accumulation, whereas equivalent concentrations of cis-platin were less effective, in line with their differential impacts on cell survival. A2780 responded to Enone-2 by time-dependent upregulation of *mdm2*, *p21, bax* and *bbc3*/*puma* mRNA, measured by RT-qPCR. Enone-1 was also found to induce p53 and p53-dependent gene expression in A2780 cells (Figure 8).

Anti-cancer agents may utilize different pathways to induce p53. For example, cis-platin induces p53 predominantly via DDR whereas oxaliplatin activates p53 through RS [11]. It has been proposed that by virtue of these differences, cis-platin and oxaliplatin "should be used in a mechanism-targeted manner for the treatment of cancer" [11] and that drugs inducing RS could particularly benefit tumors with 'translation addiction'.

To determine if the activation of p53 by Enones is mediated by DDR or RS, protein lysates isolated from A2780 cells that had been cultured for 6 hrs with increasing concentrations of either Enone-2 or cis-platin were analyzed for γH2Ax, a marker of DDR. As shown in the representative immunoblot of Figure 9A, cis-platin induced γH2Ax whereas Enone-2 had no effect relative to untreated controls. Yet, Enone-2 robustly induced p53 accumulation under the same conditions (Figure 9A). This finding suggests that p53 activation by Enones does not involve the DDR pathway.

To further validate this observation, A2780 cells were transiently transfected with siRNAs targeting Chek2 which is required for DDR-induced p53, or Rpl11 which is required for RS-mediated p53 activation [11]. The efficacy and specificity of the knockdowns were confirmed by RT-qPCR (Figure 9B). As expected [11], Chek2 knockdown ameliorated cis-platin-induced p53 activation and depletion of Rpl11 reduced oxaliplatin-induced p53 accumulation. Importantly, it was only Rpl11 knockdown that affected Enone-2 mediated p53 activation (Figures 9C & 9D). This DDR-independent mechanism of p53 activation is consistent with the observation that Enone-2 does not display cross-resistance with cis-platin in apoptosis.

We thus claim that Enone-1 and Enone-2 are inducers of the p53 pathway which is mediated by ribosomal stress pathways. Enones could thus particularly benefit tumors with 'translation addiction'.

**Experiment to prove that Enone-2 synergizes with proteasomal inhibitors in inducing cancer cell death in vitro.**

Gene expression profiling of A2780 ovarian carcinoma cells treated with 2.5 µM Enone-2 for 6 hours followed by bioinformatic analysis demonstrated enrichment of upregulated transcripts associated with proteasome-mediated ubiquitin-dependent protein catabolic process. This group entailed the following gene transcripts: ZFAND2A;KCTD10;MDM2;PLK2;DDIT3;DNAJB9;PSMD6;RYBP;PSMD1 0;SPSB3;PS MB8;PELI1;RNF103;DNAJB2;UBE2H;ATXN3;PSEN1;RCHY1;UBR1;ANAPC16;JKAM P;DDA1;PSMA6;TMUB2;TRIM72;SPOPL;UBE2J1;ARIH1;NPLOC4;CDC26;USP1 9;UF D1;TNFAIP1;PSMB6;DERL1;UBE2V2;SELENOS;PSMA1;UBE2.

Proteasome inhibitors have been reported to enhance platinum-induced toxicity in cis-platin-resistant ovarian cancer cells [12]. The significant enrichment of transcripts associated with proteasomal activity in Enone-treated cells suggested that proteasomal inhibitors may also augment Enone-2 induced cytotoxicity. To address this hypothesis, 2780CP ovarian carcinoma cells were pre-treated for 2 hours with different concentrations of the proteasomal inhibitor bortezomib, a drug in clinical use, followed by a 48 hour co-culture with Enone-2. The results demonstrated augmentation of cytotoxicity compared to each agent alone (**Figure 10**).

We thus claim that Enones could be used in combination with proteasomal inhibitors for cancer treatment. This may be particularly relevant to ovarian cancer and multiple myeloma where bortezomib has been used in the clinic.

### References

[1] Gordon AN, Fleagle JT, Guthrie D, Parkin DE, Gore ME, Lacave AJ. Recurrent epithelial ovarian carcinoma: a randomized phase III study of pegylated liposomal doxorubicin versus topotecan. J Clin Oncol. 2001;19:3312-22.
[2] Rose PG, Yao M, Chambers LM, Mahdi H, DeBernardo R, Michener CM, et al. PARP inhibitors decrease response to subsequent platinum-based chemotherapy in patients with BRCA mutated ovarian cancer. Anticancer Drugs. 2021;32:1086-92.
[3] Kalaitzakis D, Triantafyllakis M, Alexopoulou I, Sofiadis M, Vassilikogiannakis G. One-pot transformation of simple furans into 4-hydroxy-2-cyclopentenones in water. Angew Chem Int Ed Engl. 2014;53:13201-5.
[4] Ioannou GI, Montagnon T, Kalaitzakis D, Pergantis SA, Vassilikogiannakis G. Synthesis of cyclopent-2-enones from furans using a nebulizer-based continuous flow photoreactor. Org Biomol Chem. 2017;15:10151-5.
[5] Yang C, Xie N, Luo Z, Ruan X, Zhang Y, Wang W, et al. The Effect of High CDC6 Levels on Predicting Poor Prognosis in Colorectal Cancer. Chemotherapy. 2022;67:47-56.
[6] Deng Y, Jiang L, Wang Y, Xi Q, Zhong J, Liu J, et al. High expression of CDC6 is associated with accelerated cell proliferation and poor prognosis of epithelial ovarian cancer. Pathol Res Pract. 2016;212:239-46.
[7] Chen S, Chen X, Xie G, He Y, Yan D, Zheng D, et al. Cdc6 contributes to cisplatin-resistance by activation of ATR-Chk1 pathway in bladder cancer cells. Oncotarget. 2016;7:40362-76.
[8] Cai J, Wang H, Jiao X, Huang R, Qin Q, Zhang J, et al. The RNA-Binding Protein HuR Confers Oxaliplatin Resistance of Colorectal Cancer By Upregulating CDC6. Mol Cancer Ther. 2019;18:1243-54.
[9] Lim N, Townsend PA. Cdc6 as a novel target in cancer: Oncogenic potential, senescence and subcellular localisation. Int J Cancer. 2020;147:1528-34.
[10] Eliopoulos AG, Kerr DJ, Herod J, Hodgkins L, Krajewski S, Reed JC, et al. The control of apoptosis and drug resistance in ovarian cancer: influence of p53 and Bcl-2. Oncogene. 1995;11:1217-28.
[11] Bruno PM, Liu Y, Park GY, Murai J, Koch CE, Eisen TJ, et al. A subset of platinum-containing chemotherapeutic agents kills cells by inducing ribosome biogenesis stress. Nat Med. 2017;23:461-71.
[12] Huang W, Zhou Q, Yuan X, Ge ZM, Ran FX, Yang HY, et al. Proteasome Inhibitor YSY01A Enhances Cisplatin Cytotoxicity in Cisplatin-Resistant Human Ovarian Cancer Cells. J Cancer. 2016;7:1133-41.

## Claims

1. A composition comprising a 4-hydroxycyclopent-2-en-1-one derivative, for use as a medicament, **characterized in that** the said 4-hydroxycyclopent-2-en-1-one derivative has the structure: 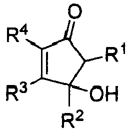 wherein
R¹ is phenyl, substituted phenyl, 2-naphthyl, substituted 1-naphthyl, -CO₂R, -H, alkyl
R² is -H, alkyl, alkenyl, benzyl
R³ is -H, alkyl
R⁴ is -H, alkyl

2. A composition comprising a 4-hydroxycyclopent-2-en-1-one derivative, for use as a medicament, according to claim 1, wherein the 4-hydroxycyclopent-2-en-1-one derivative has the structure: 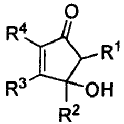 Wherein
R¹ is phenyl, 4-fluorophenyl, naphthalen-2-yl, 4-fluoronaphthalen-1-yl, CO₂Et, CO₂Me, H, n-butyl
R² is H, methyl, n-C₁₆H₃₃, benzyl, n-pentyl, n-hexyl, pent-4-enyl
R³ is H, methyl,
R⁴ is H, methyl
and preferably to have trans-stereochemistry so that the 4-hydroxy group is trans to the substituent in position-5

3. A composition comprising a 4-hydroxycyclopent-2-en-1-one derivative, for use as a medicament, according to any of the claims 1 or 2, wherein the 4-hydroxycyclopent-2-en-1-one derivative is 5-(4-fluoronaphthalen-1-yl)-4-hydroxy-4-methylcyclopent-2-en-1-one or 4-hydroxy-4-methyl-5-(naphthalen-2-yl)cyclopent-2-en-1-one and preferably to have trans-stereochemistry so that the 4-hydroxy group is trans to the substituent in position-5.

4. A composition comprising a 4-hydroxycyclopent-2-en-1-one derivative, for use as a medicament, according to any of the claims 1 to 3 wherein the said composition further comprises a delivery vehicle which enhances cellular uptake and/or stability of the compound.

5. A composition comprising a 4-hydroxycyclopent-2-en-1-one derivative, for use for treating abnormal cell growth in a mammal, **characterized in that** the said 4-hydroxycyclopent-2-en-1-one derivative has the structure: 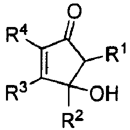 wherein
R¹ is phenyl, substituted phenyl, 2-naphthyl, substituted 1-naphthyl, -CO₂R, -H, alkyl
R² is -H, alkyl, alkenyl, benzyl
R³ is -H, alkyl
R⁴ is -H, alkyl

6. A composition comprising a 4-hydroxycyclopent-2-en-1-one derivative, for use for treating abnormal cell growth in a mammal, according to claim 5, wherein the 4-hydroxycyclopent-2-en-1-one derivative has the structure: 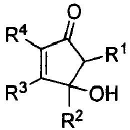 Wherein
R¹ is phenyl, 4-fluorophenyl, naphthalen-2-yl, 4-fluoronaphthalen-1-yl, CO₂Et, CO₂Me, H, n-butyl
R² is H, methyl, n-C₁₆H₃₃, benzyl, n-pentyl, n-hexyl, pent-4-enyl
R³ is H, methyl,
R⁴ is H, methyl
and preferably to have trans-stereochemistry so that the 4-hydroxy group is trans to the substituent in position-5

7. A composition comprising a 4-hydroxycyclopent-2-en-1-one derivative, for use for treating abnormal cell growth in a mammal, according to any of the claims 5 or 6, wherein the 4-hydroxycyclopent-2-en-1-one derivative is 5-(4-fluoronaphthalen-1-yl)-4-hydroxy-4-methylcyclopent-2-en-1 -one or 4-hydroxy-4-methyl-5-(naphthalen-2-yl)cyclopent-2-en-1-one and preferably the said derivatives to have trans-stereochemistry so that the 4-hydroxy group is trans to the substituent in position-5.

8. A composition comprising a 4-hydroxycyclopent-2-en-1-one derivative, for use for treating abnormal cell growth in a mammal, according to any of the claims 5 to 7, wherein the composition further comprises a proteasomal inhibitor and preferably the proteasomal inhibitor bortezomib

9. A composition comprising a 4-hydroxycyclopent-2-en-1-one derivative, for use for treating abnormal cell growth in a mammal, according to any of the claims 5 to 8, wherein the said abnormal cell growth is resistance to platinum-based chemotherapy and preferably to cis-platin treatment and/or oxaliplatin.

10. A composition comprising a 4-hydroxycyclopent-2-en-1-one derivative, for use for treating abnormal cell growth in a mammal, according to any of the claims 5 to 9, wherein the said abnormal cell growth exhibits translational addiction.

11. A composition comprising a 4-hydroxycyclopent-2-en-1-one derivative, for use for treating abnormal cell growth in a mammal, according to any of the claims 5 to 10, wherein the said abnormal cell growth is cancer and preferably cancer selected from the group consisting of basal cell cancer, medulloblastoma cancer, liver cancer, rhabdomyosarcoma, lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, myeloma, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma and pituitary adenoma, or a combination of one or more of the foregoing cancers.

12. A 4-hydroxycyclopent-2-en-1-one derivative used in the compositions of any of the previous claims 1 to 11 , with the formula

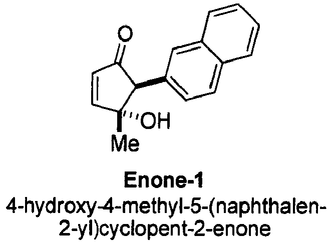

13. A 4-hydroxycyclopent-2-en-1-one derivative used in the compositions of any of the previous claims 1 to 11 , with the formula

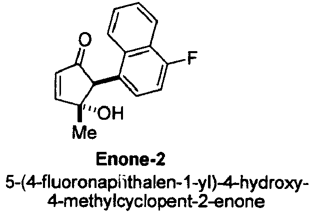

14. A 4-hydroxycyclopent-2-en-1-one derivative according to any of the claims 12 or 13 wherein the 4-hydroxy group is trans to the substituent in position-5.

15. A composition administered to a subject according to any of the claims 12-14, wherein the said pharmaceutical composition comprises a compound of the claims 1 to 11 or a tautomer or a pharmaceutically acceptable salt thereof and a delivery vehicle which enhances cellular uptake and/or stability of the compound.
